# FASCICULE DE BREVET EUROPEEN

(11) **EP 0 857 482 B1**
(45) Date de publication et mention de la délivrance du brevet: **22.05.2002**
(21) Numéro de dépôt: 98400078.6
(22) Date de dépôt: 16.01.1998
(51) Int. Cl.: A61K 9/16, A61K 33/44

(54) **Utilisation de compositions à base de charbon enrobé**
Verwendung von überzogenen Tierkohlezusammensetzungen
Use of coated charcoal compositions

(30) Priorité: 07.02.1997 FR 9701410
(43) Date de publication de la demande: 12.08.1998
(73) Titulaire: Grimberg, Georges Serge, 75005 Paris (FR)
(72) Inventeur: Grimberg, Georges Serge, 75005 Paris (FR)

(56) Documents cités:
- FR-A- 2 390 953
- US-A- 4 906 478
- LALLA J. K.; ET AL.: "Studies on dimethyl polysiloxane I: Activation of dimethicone, and evaluation and utilization of the activated product" INDIAN DRUGS, vol. 23, no. 3, 1985, pages 152-158, XP002044035

## Description

Le médicament oesogastro-intestinal représente une thérapeutique qui soigne les maladies d'origine fonctionnelle, alimentaire, bactériologique et même médicamenteuse. Si l'on considère les statistiques thérapeutiques, ce genre de médicament est souvent associé à des antispasmodiques, des tranquillisants, des vasodilatateurs cérébraux et périphériques, des analgésiques, des vasoprotecteurs, des antibiotiques, etc.

Il est traditionnellement entendu que le diméthyl polysiloxane possède une puissante action sur les gaz et que le charbon exerce une action absorbante sur ces derniers. Pourtant, l'activité thérapeutique du charbon (mis à part son activité anti-poison quand il est donné à forte dose),a toujours été contestée au point qu'aux U.S.A., et dans d'autres pays, la monographie n'a pas encore été rédigée.
En effet, une centaine de milligrammes de charbon ne peut pas absorber des litres de gaz, ni absorber sélectivement des toxines ou autres éléments perturbateurs de l'organisme.
De la même manière, l'activité thérapeutique du diméthyl polysiloxane est elle aussi très discutée.

Un médicament à base de charbon enrobé de façon à obtenir un délitement retardé actif aux différents niveaux du tube intestinal est connu du document FR-A-2 390 953.

L'objet de la présente invention est de créer un médicament oesogastro-intestinal à base de charbon enrobé d'une substance qui "reste collée au charbon" et ne contenant aucun élément en permettant le délitement, qui a une action thérapeutique totalement différente de ce qui a déjà été décrit.

Dans la présente invention, en bouchant les pores du charbon avec certains produits, on confère un pouvoir couvrant au produit ainsi créé et on obtient une activité thérapeutique beaucoup plus importante et totalement différente du problème "absorption des gaz et des toxines" ou "dégazage" gastro-intestinal.

Conformément l'invention a pour objet l'utilisation de charbon enrobé de diméthyl polysiloxane et/ou de myristate d'isopropyle pour l'obtention d'un médicament destiné à adhérer à la paroi oestogastro-intestinale.

Suivant d'autres caractéristiques de l'invention, tel que définis dans les revendications dépendantes
- le médicament à base de charbon enrobé associe d'autres principes actifs au charbon enrobé.
- le médicament à base de charbon enrobé contient plusieurs centaines de milligrammes de principes actifs, par exemple des antibiotiques, des antiacides.
- le charbon, avant d'être enrobé, est associé à des principes actifs en petites quantités, quelques dizaines de milligrammes, comme des anti-agrégants plaquettaires, des antispasmodiques, des somnifères.
- le charbon, avant ou après son enrobage, est associé à un anti-acide et à un antiseptique.
- le charbon est remplacé par de l'argile qui est enrobée et utilisée seule ou en association avec d'autres molécules.

Diverses autres caractéristiques de l'invention ressortent de la description détaillée qui suit :

Exemple n° 1 : Si on enrobe du charbon avec du diméthyl polysiloxane, on obtient un produit qui n'a plus le pouvoir absorbant du charbon pur, ni le pouvoir anti-gaz du diméthyl polysiloxane pur.
Par contre, on constate son pouvoir adhésif très puissant dans une ampoule à décanter tant en milieu aqueux que gastrique.

Une dose dite "thérapeutique" peut contenir :

| | |
|---|---|
| Charbon activé | 140 mg |
| Diméthyl polysiloxane | 45 mg |

Si l'on donne cette dose en gélule à un volontaire sain, on constate par fibroscopie que le produit adhère à la paroi stomacale.
Cette même dose peut être mise en suspension dans l'eau et son étude par fisbroscopie démontre aussi la parfaite adhérence à la paroi oesophagienne et stomacale.

Exemple n° 2 : Si on réalise un mélange de charbon et du myristate d'isopropyl, l'adhérence, là aussi, est bonne.

Exemple n° 3 : 140 mg de charbon sont donnés à un volontaire sain, on constate que l'adhérence à la paroi est totalement différente, le charbon se regroupe en morceaux. Cela de plus, est très net dans une ampoule à décanter.

Ce nouveau produit a fait l'objet d'étude :

Les indications thérapeutiques étudiées sont les suivantes :
- douleurs abdominales au sens le plus large du terme,
- troubles dyspeptiques associés avec des symptômes de : digestion lente, gêne post prandiale et somnolence, nausées, vomissements, ballonnements, météorisme,
- troubles du transit : diarrhées et/ou constipation.
Les résultats sont excellents et démonstratifs.

Donc le charbon enrobé de diméthyl polysiloxane soigne par adhérence à la paroi. Il est remarquable de constater que dans les cas "d'envie de déféquer", donc niveau intestinal, les gélules de charbon-diméthyl polysiloxane, 10 minutes après avoir été avalées, calment ce besoin, alors que le contenu de la gélule est toujours dans l'estomac. Donc l'adhérence du produit sur la surface stomacale donne "des ordres au tractus gastro-intestinal". Cela n'a donc strictement rien à voir avec le pouvoir absorbant ou anti-gaz. Il est à remarquer que les gens qui prennent ce mélange ont perdu 2 à 4 kg.

Exemple n° 4 : Le charbon est enrobé de diméthyl polysiloxane et est mélangé à un antibiotique : l'amoxiciline. Deux volontaires ont été traités à huit jours d'intervalle avec 500 mg d'amoxiciline et ensuite 500 mg d'amoxiciline et 2 gélules de charbon-diméthyl polysiloxane, formule de l'exemple n° 1.

Les résultats des prélèvements sanguins effectués au cours de 12 heures démontrent que : la quantité d'amoxiciline avec ou sans charbon enrobé est la même, mais il apparaît qu'avec le charbon enrobé, il y a un étalement des quantités dans le temps, ce qui est très favorable à l'activité du produit dans le temps.

Exemple n° 5 : L'oxyde de magnésium a une activité anti-acide parfaitement connue. Cette activité est du type neutralisant, ce qui entraîne des inconvénients quant à la durée d'action de la capacité anti-acide.
Si l'on associé de l'oxyde de magnésium à du charbon enrobé de diméthyl polysiloxane, le produit a une capacité anti-acide remarquable car l'effet dure beaucoup plus longemps grâce à l'adhérence du produit contre la paroi stomacale. Cette adhérence s'obtient aussi quand du MgO est lui aussi enrobé de diméthyl polysiloxane et associé à du charbon enrobé de diméthyl polysiloxane.

On peut aussi ajouter une molécule susceptible de détruire l'hélicobacter pyloris, ce qui permet de soigner l'ulcère gastro-duodénal ; la molécule, de l'iode par exemple, ou d'autres antiseptiques ou antibiotiques, pouvant aussi être enrobée.

Exemple n° 6 : L'acide acétyl salicylique a une activité anti-agrégante plaquettaire, si on prépare une solution d'acide acétyl salicylique dans de l'éthanol et qu'on humecte du charbon puis qu'on évapore l'éthanol, on obtient un charbon dont les pores contiennent de l'acide acétyl salycilique.
Ce charbon-acétyl salycilique enrobé avec du diméthyl polysiloxane, a été testé sur un volontaire sain.
L'activité anti-agrégante plaquettaire de ce nouveau produit est excellente et permet ainsi de diminuer la quantité d'acide acétyl salycilique donnée à l'organisme.
Donc, si les pores du charbon sont rempli par différents principes actifs puis enrobés, on obtient là encore un nouveau produit qui adhère à la paroi oesogastro-intestinale et donne, avec des petites quantités de principes actifs une bonne activité thérapeutique.

## Revendications

1. Utilisation de charbon enrobé de diméthyl polysiloxane et/ou de myristate d'isopropyle pour l'obtention d'un médicament destiné à adhérer à la paroi oesogastro-intestinale.

2. Utilisation selon la revendication 1, **caractérisé en ce que** d'autres principes actifs sont associés au charbon enrobé.

3. Utilisation selon les revendications de 1 et 2, **caractérisé en ce qu'**il contient plusieurs centaines de milligrammes de principes actifs, par exemple des antibiotiques, des antiacides.

4. Utilisation selon l'une des revendications de 1 à 3, **caractérisé en ce que** le charbon est associé, avant d'être enrobé, à des principes actifs en petites quantités, quelques dizaines de milligrammes, comme des anti-agrégants plaquettaires, des antispasmodiques, des somnifères.

5. Utilisation selon l'une des revendications de 1 à 4, **caractérisé en ce que** le charbon avant ou après enrobage, est associé à un antiacide et à un antiseptique.

6. Utilisation selon la revendication 1, **caractérisé en ce que** le charbon est remplacé par de l'argile, qui est enrobée et utilisée seule ou an association avec d'autres molécules.

## Patentansprüche

1. Benutzung von Kohle mit einer Dimethyl-polysiloxane und/oder von Isopropyle Myristate-Umhüllung zur Erhaltung eines für Wandadhäsion der Speiseröhre, des Magens oder des Darmes bestimmten Medikaments

2. Benutzung gemäss Anspruch nr. 1, dadurch kenngezeichnet, dass andere aktive Prinzipien mit Kohle zusammengesetzt werden.

3. Benutzung gemäss Anspruch nr. 1 und 2, dadurch kenngezeichnet, dass mehrere Hunderte Milligrams von aktiv Prinzipien anwesend sind, zum Beispiel Antibiotika, Antiacida.

4. Benutzung gemäss Anspruch nr. 1 zu 3, dadurch kenngezeichnet, dass Kohle und kleine Mengen von aktiven Prinzipien wieThrombozytenaggregationshemmer, Antispasmodica, Sedativa (einige Zehnte von Milligrammen) vor der Umhüllung der Kohle zusammengesetzt werden.

5. Benutzung gemäss Anspruch nr. 1 zu 4, dadurch kenngezeichnet, dass ein Antacida oder ein Antiseptika vor oder nach der Umhüllung mit dieser zusammengesetzt werden.

6. Benutzung gemäss Anspruch nr. 1, dadurch kenngezeichnet, dass Kohle durch Ton ersetzt wird, wobei Ton umgehüllt und allein oder im Zusammenstellung mit anderen Molekülen benützt wird.

## Claims

1. Use of charcoal coated with dimethyl polysiloxane and/or isopropyle myristate for obtaining a medicine intended to adhere to the oeso-gastro intestinal mucosa.

2. Use according to the claim 1, **characterized by** the fact that other active principles are associated to coated charcoal.

3. Use according to the claims 1 and 2, **characterized by** the fact that it contains several hundreds of milligrammes of active principles, e.g. antibiotics, antacids.

4. Use according to the claims from 1 to 3, **characterized by** the fact that the charcoal is associated, before to be coated, to active principles in small quantities, a few tens of milligrammes, like anti-platelets, antispasmodics, sedatives.

5. Use according to claims from 1 to 4, **characterized by** the fact that the charcoal before or after coating, is associated to an antacid and an antiseptic.

6. Use according to claim 1, **characterized by** the fact that the charcoal is replaced by clay which is coated and used alone or associated to other molecules.
